# NEUE EUROPÄISCHE PATENTSCHRIFT

(11) **EP 1 776 917 B2**
(45) Veröffentlichungstag und Bekanntmachung des Hinweises auf die Entscheidung über den Einspruch: **25.03.2015**
(45) Hinweis auf die Patenterteilung: 02.05.2012
(21) Anmeldenummer: 06021237.0
(22) Anmeldetag: 10.10.2006
(51) Int. Cl.: A61B 1/00

(54) **Endoskop**
Endoscope
Endoscope

(30) Priorität: 18.10.2005 DE 102005051209
(43) Veröffentlichungstag der Anmeldung: 25.04.2007
(73) Patentinhaber: Karl Storz GmbH & Co. KG, 78532 Tuttlingen (DE)
(72) Erfinder: Fürst, Frank, 78532 Tuttlingen (DE); Halbig, Jörg, 78467 Konstanz (DE); Kupferschmid, Markus, 78576 Emmingen-Liptingen (DE); Lei, Fang, 78591 Durchhausen (DE); Mersmann, Sven, 78532 Tuttlingen (DE); Dahmen, Jan, 78606 Seitigen-Oberflacht (DE); Stihl, Ewald, 78187 Geisingen (DE); Lederer, Frank, 78532 Tuttlingen (DE)
(74) Vertreter: Heuckeroth, Volker

(56) Entgegenhaltungen:
- EP-A1- 1 523 932
- DE-A1- 3 740 417
- DE-A1- 4 211 547
- DE-A1- 10 307 903
- DE-A1-102004 008 458
- US-A- 3 261 350
- US-A- 4 601 713
- US-A- 4 790 295
- US-A- 4 921 479
- US-A- 5 051 824
- US-A- 5 735 792
- US-A1- 2005 182 387
- 'Meyers Enzyklopädisches Lexikon', 1980, BIBLIOGRAPHISCHES INSTITUT, MANNHEIM Seite 146
- Wikipedia: "Balkentheorie", gefunden 23.01.2013
- DR.-ING. I. SZABÓ: 'Einführung in die Technische Mechanik', 1963, SPRINGER-VERLAG, BERLIN - GÖTTINGEN - HEIDELBERG Seiten 36-37 - 204
- Auszug aus "Ernst Wörterbuch der industriellen Technik"
- Deutsche Übersetzung des Begriffs "rigid" gemäß Webseite http://www.leo.org/.

## Beschreibung

Die Erfindung betrifft ein Endoskop, mit einem Schaft, der ein äußeres Rohr und ein darin verlaufendes inneres Rohr aufweist, wobei das äußere und das innere Rohr radial voneinander beabstandet sind, so dass diese zwischen sich einen sich axial erstreckenden Kanal bilden, in dem ein Lichtleiter aufgenommen ist.

Derartige Endoskope sind aus dem DE-Katalog der Karl Storz GmbH & Co. KG ("Arthroskopie und Sportmedizin-Wirbelsäule" (02/2003), Seite ART-TEL 4, Instrument 28731 BWA) bekannt.

Solche Endoskope werden beispielsweise in minimal-invasiven Eingriffen in der Arthroskopie, in der Bauch- und Brustkorbspiegelung, bei Leistenbrüchen sowie bei Gelenk- und Wirbelsäulenoperationen eingesetzt. Dabei unterstützen die Endoskope den Operateur bei der Durchführung der Operation, indem sie einen möglichst großen Sichtbereich im Operationsgebiet einsehbar machen.

Während solcher Operationen müssen diese Instrumente durch äußerst kleine Zugänge in das Operationsgebiet eingeführt werden können. Dazu bedarf es möglichst schlanker Instrumentarien.

Ein solches Endoskop weist einen länglichen Schaft auf. Dieser längliche Schaft weist zwei ineinander geschobene Rohre auf. Dabei ist der Durchmesser des inneren Rohres so gewählt, dass dieses von dem äußeren Rohr radial beabstandet ist. Dadurch wird zwischen dem inneren und dem äußeren Rohr ein Kanal gebildet. Dieser Kanal dient der Aufnahme eines Lichtleiters, der durch eine Vielzahl von Glasfasern gebildet ist.

Ein distales Ende des Schaftes ist angeschrägt und eine entsprechend bearbeitete Linse ist distal eingepasst. Diese Anordnung gewährt durch die Linse einen Blickwinkel von beispielsweise 30° oder 70°, bezogen auf die Mittellängsachse des Endoskops, auf das Operationsgebiet.

Der Schaft ist mit seinem proximalen Ende in einem Endoskopkopf aufgenommen. Dabei kann sich das innere Rohr bis in eine Innenbuchse des Endoskopkopfes hinein erstrecken. Das äußere Rohr des Schaftes endet proximal so im Endoskopkopf, dass der Kanal durch einen Hohlraum zu einem Lichtleiteranschluss im Endoskopkopf führt.

Dieser Lichtleiteranschluss ist quer zur Längsachse des Schaftes im Endoskopkopf eingebracht und weist eine zentrale Öffnung auf. Somit erstreckt sich der Kanal ununterbrochen vom distalen zum proximalen Ende des Schaftes.

Durch einen in dem Kanal angeordneten Lichtleiter, z. B. ein Glasfaserbündel, ist es daher möglich, durch diesen Kanal Licht zu leiten, um das Operationsgebiet auszuleuchten.

Am Endoskopkopf kann weiterhin eine Okularmuschel angebracht sein, so dass ein Operateur durch die Okularmuschel und den Schaft bequem das Operationsgebiet beobachten kann. Für diesen Zweck ist im inneren Rohr ein Satz von Linsen bzw. Stablinsen angeordnet. Anstelle eines Okulars zur direkten Beobachtung durch das Endoskop kann am proximalen Ende auch eine Videokamera vorgesehen sein, deren Bildsignale auf einem Monitor dargestellt werden.

Während einer Operation kann es vorkommen, dass der Operateur die Position des Endoskops verändern muss, um die Operationsstelle aus einem anderen Blickwinkel beobachten zu können. Unter Umständen können dabei Kräfte seitlich bzw. schräg auf den Schaft des Endoskops einwirken, so dass sich dieser etwas verbiegt. Dabei ist besonders der proximale Teil des Schaftes von einer Verbiegung betroffen.

Es ist dabei als besonders nachteilig anzusehen, dass durch eine stärkere Biegung des Schaftes Brüche der Stablinsen auftreten können. Aber auch bereits weniger starke Verbiegungen können ausreichend sein, um ein Brechen der im Kanal befindlichen Glasfasern hervorzurufen. Eine solche Beschädigung des Endoskops bedeutet zumindest eine eingeschränkte Funktion durch eine Schwächung der Lichtleitung. Sollte darüber hinaus eine Stablinse zerbrechen, bedeutet dies eine völlige Unbrauchbarkeit des Endoskops. Eine Beschädigung dieser Art ist während einer Operation besonders von großem Nachteil.

Aus dem Dokument DE 103 07 903 A1 ist ein Verfahren zum Montieren eines Endoskops sowie ein derartiges Endoskop bekannt. Der Schaft dieses bekannten Endoskops weist ein äußeres Rohr auf, in dem ein inneres Rohr zur Aufnahme der Bildoptik angeordnet ist. Die zwischen dem inneren und dem äußeren Rohr angeordneten Lichtleiter sind in einem flexiblen Schlauch aufgenommen.

In dem Dokument US 4,790,295 ist ein Endoskop beschrieben, dessen Schaft ein äußeres Rohr aufweist. Innerhalb des äußeren Rohrs ist ein inneres Rohr zur Durchführung von Zangen angeordnet. Ferner ist innerhalb des äußeren Rohrs distalseitig ein kurzes, als Linsenrahmen dienendes Zwischenrohr aufgenommen. In dem Linsenrahmen sind bildleitende Elemente angeordnet. Die Lichtleiter sind zwischen dem inneren und dem äußeren Rohr angeordnet. Das als Linsenrahmen ausgebildete Zwischenrohr umgibt dabei nicht das innere Rohr.

Aus dem Dokument DE 10 2004 008 458 A1 ist ein Verfahren zum Fixieren von Glasfasern in einem Endoskop bekannt. Der Schaft dieses Endoskops weist ein äußeres Rohr auf. Innerhalb des äußeren Rohrs ist ein als Optikrohr ausgebildetes inneres Rohr aufgenommen, in dem bildleitende Elemente angeordnet sind. Zwischen beiden Rohren verlaufen zur Lichtleitung Glasfaserbündel.

Aus dem Dokument US 5,735,792 ist ein Endoskop bekannt, dessen Schaft ein äußeres Rohr und ein inneres Rohr aufweist, wobei das äußere Rohr und das innere Rohr unmittelbar aneinander anliegen. Das äußere Rohr und das innere Rohr sind aus Edelstahl gefertigt, um eine hohe Steifigkeit des Endoskopschaftes zu erreichen. In dem inneren Rohr ist eine optische Anordnung in einer Hülle aufgenommen, wobei die optische Anordnung auch einen Lichtleiter aufweist. Zwischen dem inneren Rohr und der Hülle ist ein Kanal vorhanden, der als Spülkanal dient.

Schließlich ist aus dem Dokument DE 199 28 289 A1 ein Endoskop bekannt, dessen Schaft ein äußeres Rohr aufweist. Innerhalb des äußeren Rohrs sind zwei innere Rohre, und zwar ein Arbeitsrohr für Spülflüssigkeiten und ein Optikrohr zur Auf-nahme von bildleitenden Elementen, angeordnet. Zwischen dem Optikrohr, dem Arbeitsrohr und dem äußeren Rohr sind Lichtleitfasern zur Lichtleitung angeordnet.

Bei allen vorstehend beschriebenen bekannten Endoskopen besteht der Nachteil fort, dass die Glasfasern des Lichtleiters oder Linsen nicht hinreichend gegen Bruch geschützt sind.

Es ist daher Aufgabe der vorliegenden Erfindung, ein Endoskop der eingangs genannten Art dahingehend weiterzubilden, dass der Zusammenbau des Schaftes erleichtert wird.

Erfindungsgemäß wird diese Aufgabe dadurch gelöst, dass das Zwischenrohr einen axialen Schlitz aufweist.

Durch das biegesteife Zwischenrohr, das sich im Schaft zwischen dem äußeren Rohr und dem inneren Rohr erstreckt, wird der Schaft vorteilhafterweise verstärkt. Eine Materialverstärkung dieser Art führt zu einer erhöhten Stabilität des Schaftes. Seitlich bzw. schräg am Schaft angreifende Kräfte führen daher nicht mehr oder zumindest nur in einem geringeren Ausmaß zu einer Verbiegung des Schaftes. Dabei erfüllt der so verstärkte Schaft zwei Schutzfunktionen. Zum einen werden im Schaft befindliche Stablinsen vor einem Ausbrechen bzw. vor einem Zerbrechen durch angreifende Verbiegungskräfte geschützt. Zum anderen wird ein Lichtleiter, der sich im Kanal zwischen dem inneren Rohr und dem äußeren Rohr erstreckt, ebenso vor angreifenden Verbiegungskräften geschützt. Es wird hierdurch einer Beschädigung des Lichtleiters vorgebeugt. Da das Zwischenrohr zwischen innerem und äußerem Rohr angeordnet ist, wird eine Außendurchmesservergrößerung des Schafts vorteilhaft vermieden.

In einer bevorzugten Ausgestaltung ist das Zwischenrohr kürzer als das äußere Rohr oder das innere Rohr.

Diese Maßnahme hat den Vorteil, dass durch ein kürzeres Zwischenrohr ein ebenfalls verstärkter Schaft herstellbar ist, der an besonders beanspruchten Stellen des Endoskops verstärkt ist, dabei aber eine Materialersparnis im Vergleich zu einem ungekürzten Zwischenrohr aufweist. Das verkürzte Zwischenrohr erleichtert weiterhin den Zusammenbau eines Endoskopschaftes dahingehend, dass ein Verhaken oder Verkanten des Zwischenrohres aufgrund einer besseren Handhabbarkeit kürzerer Teile während des Zusammenbaus leichter korrigiert werden kann.

In einer weiteren bevorzugten Ausgestaltung der Erfindung ist das Zwischenrohr in einem proximalen Bereich des Schaftes angeordnet.

Diese Maßnahme hat den Vorteil, dass das Zwischenrohr zur Verstärkung in demjenigen Bereich des Schaftes angeordnet ist, der durch seitlich bzw. schräg angreifende Kräfte am stärksten einer Belastung ausgesetzt ist und am ehesten dazu neigt, sich zu verbiegen. Dabei auftretende Spannungen können, wie oben erwähnt, zu einem Bruch des Lichtleiters bzw. der Stablinsen führen, was nunmehr vermieden werden kann. Eine proximal angeordnete Verstärkung erschwert bzw. verhindert ein Verbiegen des Schaftes in diesem Bereich und schützt daher die genannten Elemente gut vor einer Beschädigung.

Das Zwischenrohr weist einen axialen Schlitz auf.

Dies hat den Vorteil, dass das Zwischenrohr beim Zusammenbau des Schaftes einfacher zwischen die beiden anderen Rohre eingeschoben werden kann. Durch ein radiales Zusammendrücken des geschlitzten Zwischenrohres ist ein Ver-kanten oder Verkeilen des Zwischenrohres während des Einschiebens nahezu unmöglich bzw. einfach korrigierbar. Sollte sich das Zwischenrohr während des Einschiebens einmal verhaken, so kann durch ein leichtes Zusammendrücken des Zwischenrohres dieser Widerstand überwunden und das Zwischenrohr an dem Hindernis vorbei weiter eingeschoben werden. Dies ist bis zum vollständigen Einschub des Zwischenrohres möglich und erleichtert auf diese Weise den Zusammenbau des Schaftes erheblich.

Darüber hinaus hat diese Maßnahme den Vorteil, dass sich das Zwischenrohr auf-grund entsprechend gewählter Abmessungen von innen an das äußere Rohr sehr gut anschmiegt. Im praktischen Einsatz des Endoskopes wird dadurch verhindert, dass sich das Zwischenrohr zwischen dem äußeren und dem inneren Rohr hin- und herbewegen kann.

In einer weiteren bevorzugten Ausgestaltung der Erfindung ist das Zwischenrohr an seinem proximalen Ende angeschrägt.

Diese Maßnahme hat den Vorteil, dass ein Bereich im proximalen Ende des Schaftes vom Zwischenrohr ausgespart bleibt. Das Einführen des Lichtleiters in den Kanal, d.h. in die Beabstandung zwischen innerem und äußerem Rohr, lässt sich aufgrund der Aussparung spannungsfrei und frei von Knicken an dem Zwischenrohr vorbei durchführen. Auf diese Weise wird verhindert, dass der Lichtleiter am proximalen Ende des Zwischenrohres aufliegt oder geknickt wird und deshalb Gefahr läuft, an diesem Ende abzubrechen.

In einer weiteren bevorzugten Ausgestaltung der Erfindung weist der Schaft, radial von innen nach außen gesehen, das innere Rohr, das Zwischenrohr, den Kanal und das äußere Rohr auf.

Diese Maßnahme hat den Vorteil, dass durch diese Anordnung der Rohre das innere Rohr eine zusätzliche Verstärkung erfährt. Dadurch erhöht sich die Stabilität des Schaftes und ein Verbiegen des Schaftes tritt nur bei einer entsprechend erhöhten einwirkenden Biegekraft auf. Somit erfahren die im Innenrohr angeordneten Stablinsen insgesamt einen verbesserten Schutz durch den Schaft. Die Wahrscheinlichkeit, dass die Stablinsen ausplatzen oder zerbrechen ist dadurch sehr viel geringer, als in einem unverstärkten Schaft.

In einer weiteren bevorzugten Ausgestaltung der Erfindung weist der Schaft, radial von innen nach außen gesehen, das innere Rohr, den Kanal, das Zwischenrohr und das äußere Rohr auf.

Diese Maßnahme hat den Vorteil, dass durch diese Anordnung der Rohre sowohl die Stablinsen im inneren Rohr als auch der Kanal zur Aufnahme des Lichtleiters durch die Verstärkung des Schaftes besser geschützt sind. Ein Verbiegen des Schaftes tritt erst bei höheren Kräften als bei einem ungeschützten Schaft auf. Dies verhindert, wie schon vorher genannt, sowohl ein Ausbrechen bzw. ein Zerbrechen der Stablinsen als auch eine Beschädigung des Lichtleiters durch eine Verbiegung des Schaftes.

In einer weiteren bevorzugten Ausgestaltung der Erfindung ist das Zwischenrohr hinsichtlich seiner Länge und Wandstärke so gestaltet, dass das Zwischenrohr nur einen Raum zwischen dem äußeren Rohr und dem inneren Rohr ausfüllt, der vom Lichtleiter freigelassen ist.

Diese Maßnahme hat den Vorteil, dass beim Einbringen des Zwischenrohres in den Kanal darauf geachtet wird, dass der Raum für den Lichtleiter im Kanal in seinen Abmessungen unverändert belassen wird. Folglich gilt, dass auch der für eine Aufnahme vorgesehene Lichtleiter seine Abmessungen unverändert beibehält. Somit sind über den gesamten Kanal unveränderte Lichtleitereigenschaften gewährt.

Es ergibt sich daher eine durch das Zwischenrohr vollständig unbeeinflusste Lichtleitung und damit eine gleichbleibende Ausleuchtung des Operationsgebietes. Einem Operateur steht somit ein verstärktes, stabileres Endoskop mit denselben Lichtleitungseigenschaften im Vergleich zu einem unverstärkten Endoskop für eine Operation zur Verfügung.

In einer weiteren bevorzugten Ausgestaltung der Erfindung ist distal auf das innere Rohr ein teilumfängliches Segment aufgeschoben, das im Wesentlichen zwischen dem äußeren Rohr und dem inneren Rohr angeordnet ist.

Diese Maßnahme hat den Vorteil, dass ein Abstandshalter in einem Raum zwischen dem inneren und dem äußeren Rohr eingebracht ist. Dies stellt sicher, dass das Zwischenrohr im praktischen Einsatz am distalen Ende seine Position beibehält. Die angeordneten Stablinsen sind damit vor einem Verrutschen durch ein Verbiegen des Schaftes geschützt. Ein Aufrechterhalten der optischen Eigenschaften des Endoskopes im praktischen Einsatz ist damit gewährleistet.

In einer weiteren bevorzugten Ausgestaltung der Erfindung weist das teilumfängliche Segment eine Linse auf.

Diese Maßnahme hat den Vorteil, dass zusammen mit einem austauschbar angebrachten Segment am distalen Ende des Schaftes die distal angebrachte Linse des Endoskops austauschbar ist. Da diese Linse im Operationsgebiet distalseitig besonderen Beanspruchungen bzw. einem Verschleiß ausgesetzt ist, ist es vorteilhaft, diese Linse nach einer gewissen Zeit austauschen und auf diese Weise das Endoskop wieder instand setzen zu können. Auf diese Weise ist es unnötig, das Endoskop nach einem Verschleiß dieser einen Linse komplett austauschen zu müssen. Eine Reduktion des Austausches auf Verschleißteile bedeutet eine Material- und Kostenersparnis.

In einer weiteren bevorzugten Ausgestaltung ist das teilumfängliche Segment an seinem distalen Ende angeschrägt.

Diese Maßnahme hat den Vorteil, dass im distalen Bereich des Schaftes der Lichtleiter um das teilumfängliche Segment herum angeordnet werden kann. Dies ermöglicht eine Schrägstellung des Lichtaustritts bezogen auf die Mittellängsachse des Endoskops. Somit ergibt sich eine Ausleuchtung des Operationsgebietes in einem entsprechenden Winkel. In Kombination mit einer entsprechend angeschrägten Linse erweitert dies das Sichtfeld des Operateurs im Operationsgebiet erheblich.

In einer weiteren bevorzugten Ausgestaltung ist der Lichtleiter ein Glasfaserbündel.

Diese Maßnahme hat den Vorteil, dass selbst bei Ausfall einer kleineren Menge von Glasfasern in diesem Bündel immer noch eine annehmbare Ausleuchtung des Operationsgebietes gewährleistet ist.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand eines ausgewählten Ausführungsbeispiels im Zusammenhang mit den beiliegenden Zeichnungen näher beschrieben und erläutert. Es zeigen:
- Fig. 1: eine perspektivische Ansicht eines erfindungsgemäßen Endoskopes;
- Fig. 2: ein Schnitt durch ein erfindungsgemäßes Endoskop mit einem Endoskopkopf und einem unterbrochenen Schaft;
- Fig. 3: ein unterbrochen und vergrößert dargestelltes distales Ende eines Schaftes; und
- Fig. 4: ein Schnitt entlang der Linie IV-IV der Fig. 3.

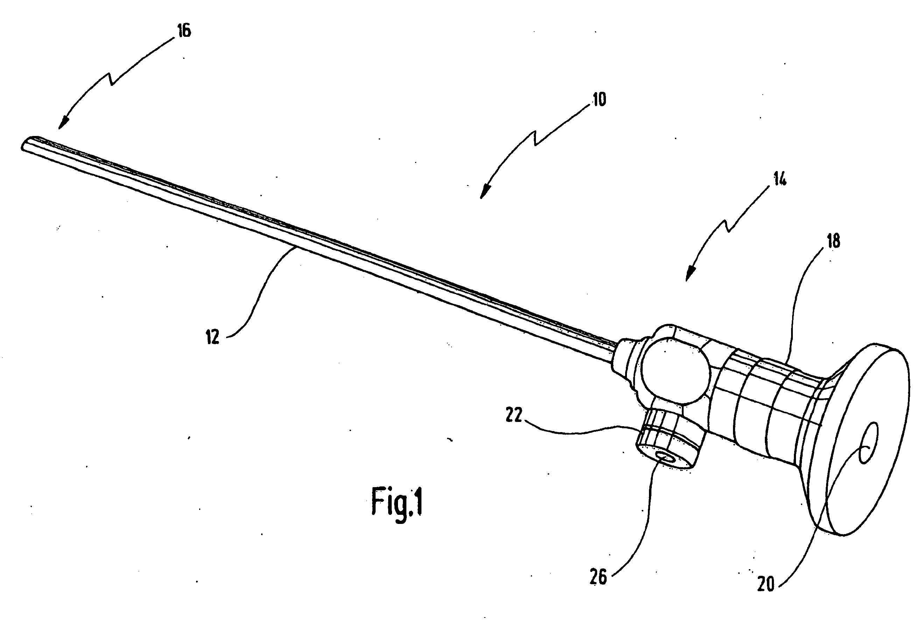

In Figur 1 ist ein Endoskop dargestellt, dass in seiner Gesamtheit mit der Bezugsziffer 10 bezeichnet ist.

Dieses Endoskop 10 weist einen Schaft 12 auf, der ein proximales Ende 14 und ein distales Ende 16 aufweist.

Ferner ist der Schaft 12 mit seinem proximalen Ende 14 in einem Endoskopkopf 18 aufgenommen.

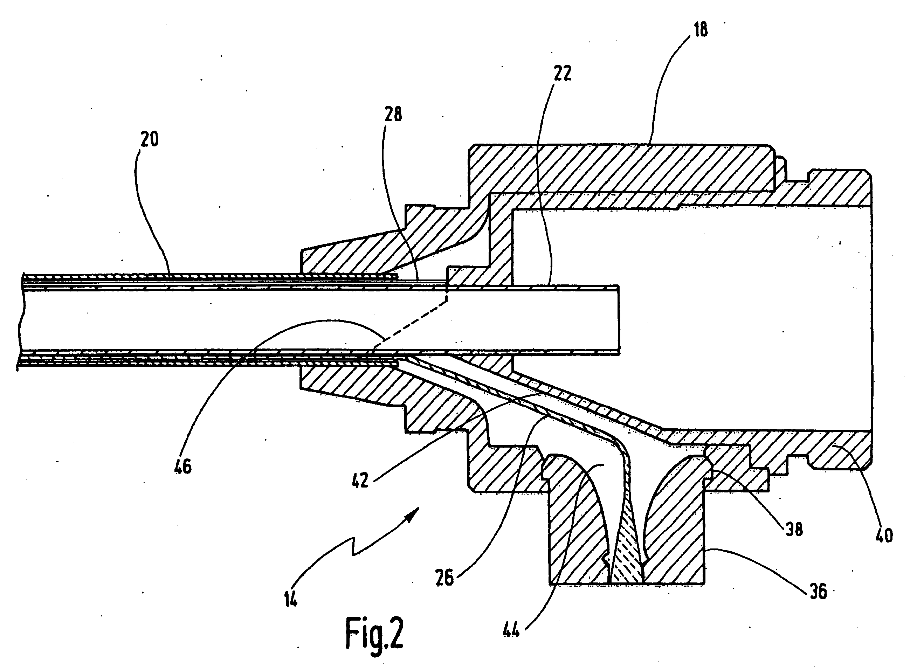

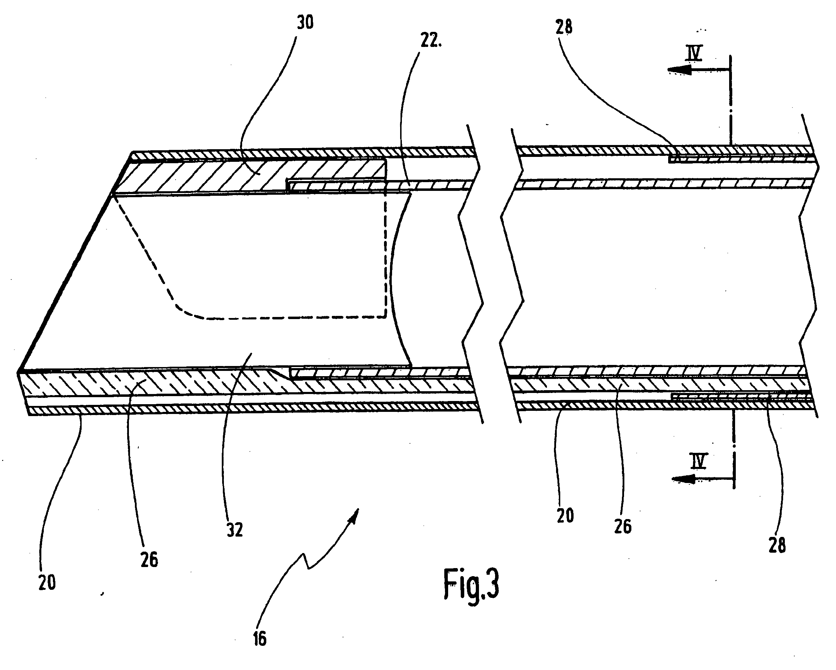

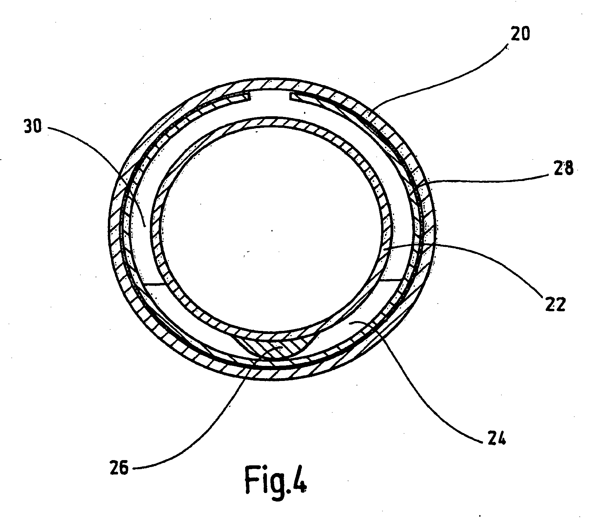

In der schematischen Figur 2 sowie in den Figuren 3 und 4 ist ersichtlich, dass der Schaft 12 aus mehreren Teilen aufgebaut ist.

Der Schaft 12 weist ein äußeres Rohr 20 auf. In diesem äußeren Rohr 20 verläuft ein durchmessergeringeres inneres Rohr 22. Dabei sind diese beiden Rohre 20 und 22 so voneinander beabstandet, dass sie zusammen einen Kanal 24 bilden. Figur 4 veranschaulicht, dass dieser Kanal 24 genügend Raum für einen in ihm geführten Lichtleiter 26 bietet.

Darüber hinaus sind das äußere Rohr 20 und das innere Rohr 22 so beabstandet, dass im Kanal 24 neben dem Lichtleiter 26 weiterhin noch ein freier Raum vorhanden ist, der in dem erfindungsgemäßen Endoskop 10 durch das Einfügen eines Zwischenrohres 28 genutzt wird.

Wie in Figur 2 dargestellt, ist das Zwischenrohr 28 kürzer als das äußere Rohr 20 bzw. das innere Rohr 22. Ferner ist das Zwischenrohr 28 in einem proximalen für Biegungskräfte anfälligen Bereich des Schaftes 12 angeordnet. Es weist darüber hinaus einen axialen Schlitz auf, der es erlaubt das Zwischenrohr 28 radial zusammenzudrücken. In der Figur 2 ist ferner gestrichelt dargestellt, dass das Zwischenrohr 28 an seinem proximalen Ende angeschrägt ist.

In der in Figur 2 angegebenen bevorzugten Ausgestaltung des Endoskopes 10 ist dargestellt, dass das Zwischenrohr 28 so im Schaft 12 angeordnet ist, dass der Schaft 12, radial von innen nach außen gesehen, das innere Rohr 22, den Kanal 24, das Zwischenrohr 28 und das äußere Rohr 20 aufweist.

In dieser Anordnung schmiegt sich das Zwischenrohr 28 innenwandig an das äußere Rohr 20 an.

Es ist aber auch eine weitere hier nicht dargestellte Anordnung denkbar, in der der Schaft 12, radial von innen nach außen gesehen, das innere Rohr 22, das Zwischenrohr 28, den Kanal 24 und dann das äußere Rohr 20 aufweist.

In dieser Anordnung liegt das Zwischenrohr 28 außenwandig am inneren Rohr 20 eng an, so dass der Kanal 24 für den Lichtleiter 26 erhalten bleibt.

Wichtig für die Funktion des Endoskopes 10 ist dabei insgesamt, dass das Zwischenrohr 28 nur einen Raum zwischen dem äußeren Rohr 20 und dem inneren Rohr 22 ausfüllt, der vom Lichtleiter 26 freigelassen ist.

Die Funktion des Zwischenrohres 28 wird weiter unten beschrieben.

Der Schaft 12 ist, wie aus den Figuren 1 und 3 ersichtlich, an seinem distalen Ende 16 angeschrägt. Ferner ist in Figur 3 gezeigt, dass im distalen Ende 16 des Schaftes 12 ein Segment 30 vorgesehen ist. Dieses Segment 30 weist an seinem distalen Ende eine Schräge auf. Das Segment 30 dient der Halterung einer Linse 32. Die Linse 32 ist der Schräge des distalen Endes 16 entsprechend geformt und bewirkt durch ihre Brechkraft für das Endoskop 10 eine veränderte Blickrichtung und ein erweitertes Gesichtsfeld.

Das Segment 30 weist beispielsweise eine Länge von etwa 1 bis 2 cm auf. Es weist in etwa die Form eines Halbrohres auf und verläuft teilumfänglich um das innere Rohr 22, wobei es in das äußere Rohr 20 mit seinen Abmessungen eingepasst ist. Auf diese Weise füllt es den Abstand zwischen den beiden Rohren 20 und 22 aus und sitzt formschlüssig zwischen ihnen.

Dabei weist das Segment 30 innenwandig eine Schulter auf, die so ausgebildet ist, dass das Segment 30 passend auf das innere Rohr 22 aufgeschoben werden kann und zu Liegen kommt. Dadurch wird die Einschubtiefe des Segments 30 in das äußere Rohr 20 begrenzt.

Die Form des Segments 30 ist seinen Funktionen angepasst. Zum einen dient das Segment 30 als Abstandshalter zwischen dem äußeren Rohr 20 und dem inneren Rohr 22, zum anderen dient es als Fassung für die Linse 32.

Gemäß dem Einsatzzweck des Endoskops 10 können beispielsweise Blickwinkel um 30° oder 70°, bezogen auf die Mittellängsachse des Endoskopes 10 eingestellt sein.

Das Endoskop 10 weist ferner den Endoskopkopf 18 auf.

Dieser Endoskopkopf 18 ist ebenfalls mehrteilig aufgebaut. In der Figur 1 ist gezeigt, dass er proximal mit einer Okularmuschel 34 versehen ist, durch die ein Operateur in einem endoskopischen Eingriff ein zu untersuchendes Operationsfeld einsehen kann.

Es ist aber auch denkbar am Endoskopkopf 18 eine Videokamera anzuschließen und aufgenommene Bildsignale auf einen Monitor zu übertragen oder zu digitalisieren und per Computer zu analysieren.

Ferner weist der Endoskopkopf 18 einen Lichtleiteranschluss 36 auf. Dieser Lichtleiteranschluss 36 ist, wie aus Figur 2 ersichtlich, von innen in eine Fassung 38 eingesetzt. Der Lichtleiteranschluss 36 weist eine Öffnung auf. In dieser Öffnung ist der Lichtleiter 26 aufgenommen. Im Bereich des Ausgangs der Öffnung wurde diese sowie der Lichtleiter 26 aus Gründen der leichteren zeichnerischen Darstellbarkeit vergrößert dargestellt.

Im Endoskopkopf 18 ist ferner ein Halterungselement 40 angeordnet. Dieses Halterungselement 40 ist in der Figur 2 als eine Hülse dargestellt, die eine Öffnung in ihrem Boden aufweist.

Proximal weist das Halterungselement 40 eine Schulter auf, mit der es als Einsatz im Endoskopkopf 18 formschlüssig aufliegt. Damit ist der Endoskopkopf 18 proximal abgedichtet.

Nach distal gerichtet weist das Halterungselement 40 eine Verjüngung 42 auf, die sich zur Öffnung im Boden hin erstreckt. Mit dieser Öffnung nimmt das Halterungselement 40 das innere Rohr 22 festsitzend im Endoskopkopf 18 auf.

Insgesamt ist das Halterungselement 40 durch die Verjüngung 42 so gestaltet, dass sich im Endoskopkopf 18 ein Hohlraum 44 bildet. Dieser Hohlraum 44 schließt sich proximal an den Kanal 24 an und erweitert diesen in den Endoskopkopf 18 hinein. Durch diesen Hohlraum 44 ist der Lichtleiter 26 zum Lichtleiteranschluss 36 geführt.

Wie bereits weiter oben beschrieben und aus den Figuren 2 bis 4 ersichtlich, ist zwischen dem äußeren und dem inneren Rohr 20 und 22 ein Zwischenrohr 28 angeordnet.

Dieses Zwischenrohr 28 sitzt mit seinem proximalen Ende auf dem Halterungselement 40 auf. Dabei weist es proximal eine Schräge 46 auf, so dass es nur mit einer verringerten Rohrkante auf dem Halterungselement 40 aufsitzt.

Diese Ausgestaltung des Zwischenrohres 28 ist vorteilhaft, wenn der Lichtleiter 26 vom Lichtleiteranschluss 36 durch den Hohlraum 44 in den Kanal 24 zum distalen Ende 16 des Schaftes 12 geführt ist.

Als Lichtleiter 26 eignet sich insbesondere ein Glasfaserbündel. Ein solches Glasfaserbündel wird auch in der hier dargestellten und beschriebenen Erfindung bevorzugt als Lichtleiter 26 verwendet.

Ferner weist das Zwischenrohr 28 einen Schlitz auf, der sich parallel zur Längsachse vollständig durch das Zwischenrohr 28 erstreckt. Dabei wird das Zwischenrohr 28 bevorzugt so in den Schaft 12 eingesetzt, dass der Schlitz diametral gegenüber dem Lichtleiter 26 zu Liegen kommt, wie aus der Figur 4 hervorgeht.

Sollte sich während des Zusammenbaus des Schaftes 12 das Zwischenrohr 28 einmal im äußeren Rohr 20 verkanten oder verhaken, kann das Zwischenrohr 28 aufgrund des Schlitzes etwas zusammengedrückt und am Hindernis vorbei weiter eingeschoben werden.

Es versteht sich, dass das Zwischenrohr vorteilhafterweise aus einem Material mit hoher Biegesteifigkeit, insbesondere aus für medizinische Instrumente dieser Art verwendetem Stahl oder beispielsweise aus metallischen Legierungen, wie beispielsweise Monel, Phynox (Kobaltlegierung), hergestellt ist.

Insgesamt ergibt sich für das Endoskop 10 ein stabiler Schaft 12, der bevorzugt an den Stellen verstärkt ist, die im praktischen Einsatz des Endoskops 10 besonders durch seitlich oder schräg angreifende Kräfte beansprucht werden.

## Patentansprüche

1. Endoskop, mit einem Schaft (12), der ein äußeres Rohr (20) und ein darin verlaufendes inneres Rohr (22) aufweist, wobei das äußere und das innere Rohr (20, 22) radial voneinander beabstandet sind, so dass diese zwischen sich einen sich axial erstreckenden Kanal (24) bilden, in dem ein Lichtleiter (26) aufgenommen ist, **dadurch gekennzeichnet, dass** zwischen dem äußeren Rohr (20) und dem inneren Rohr (22) weiterhin ein biegesteifes Zwischenrohr (28) angeordnet ist, das das innere Rohr (22) zumindest teilweise umgibt, wobei das Zwischenrohr (28) einen axialen Schlitz aufweist.

2. Endoskop nach Anspruch 1, **dadurch gekennzeichnet, dass** das Zwischenrohr (28) kürzer ist als das äußere Rohr (20) oder das innere Rohr (22).

3. Endoskop nach Anspruch 1 oder 2, **dadurch gekennzeichnet, dass** das Zwischenrohr (28) in einem proximalen Bereich des Schaftes (12) angeordnet ist.

4. Endoskop nach einem der Ansprüche 1 bis 3, **dadurch gekennzeichnet, dass** das Zwischenrohr (28) an seinem proximalen Ende angeschrägt ist.

5. Endoskop nach einem der Ansprüche 1 bis 4, **dadurch gekennzeichnet, dass** der Schaft (12), radial von innen nach außen gesehen, das innere Rohr (22), das Zwischenrohr (28), den Kanal (24) und das äußere Rohr (20) aufweist.

6. Endoskop nach einem der Ansprüche 1 bis 4, **dadurch gekennzeichnet, dass** der Schaft (12), radial von innen nach außen gesehen, das innere Rohr (22), den Kanal (24), das Zwischenrohr (28) und das äußere Rohr (20) aufweist.

7. Endoskop nach einem der Ansprüche 1 bis 6, **dadurch gekennzeichnet, dass** das Zwischenrohr (28) hinsichtlich seiner Länge und Wandstärke so gestaltet ist, dass das Zwischenrohr (28) nur einen Raum zwischen dem äußeren Rohr (20) und dem inneren Rohr (22) ausfüllt, der vom Lichtleiter (26) freigelassen ist.

8. Endoskop nach einem der Ansprüche 1 bis 7, **dadurch gekennzeichnet, dass** distal auf das innere Rohr (22) ein teilumfängliches Segment (30) aufgeschoben ist, das im Wesentlichen zwischen dem äußeren Rohr (20) und dem inneren Rohr (22) angeordnet ist.

9. Endoskop nach Anspruch 8, **dadurch gekennzeichnet, dass** das teilumfängliche Segment (30) eine Linse (32) aufweist.

10. Endoskop nach Anspruch 8 oder 9, **dadurch gekennzeichnet, dass** das teilumfängliche Segment (30) an seinem distalen Ende angeschrägt ist.

11. Endoskop nach einem der Ansprüche 1 bis 10, **dadurch gekennzeichnet, dass** der Lichtleiter (26) ein Glasfaserbündel ist.

## Claims

1. An endoscope, comprising a shaft (12) having an outer tube (20) and an inner tube (22) extending inside the latter, the outer and inner tubes (20, 22) being radially spaced apart from one another such that they form between them an axially extending channel (24) in which a light guide (26) is accommodated, **characterized in that** an intermediate tube (28) having flexural strength is arranged between the outer tube (20) and the inner tube (22), which intermediate tube (28) at least partially surrounds the inner tube (22), the intermediate tube (28) having an axial slit.

2. The endoscope of claim 1, **characterized in that** the intermediate tube (28) is shorter than the outer tube (20) or the inner tube (22).

3. The endoscope of claim 1 or 2, **characterized in that** the intermediate tube (28) is arranged in a proximal area of the shaft (12).

4. The endoscope of anyone of claims 1 through 3, **characterized in that** the intermediate tube (28) is bevelled at its proximal end.

5. The endoscope of anyone of claims 1 through 4, **characterized in that** the shaft (12), viewed radially from the inside outward, comprises the inner tube (22), the intermediate tube (28), the channel (24), and the outer tube (20).

6. The endoscope of anyone of claims 1 through 4, **characterized in that** the shaft (12), viewed radially from the inside outward, comprises the inner tube (22), the channel (24), the intermediate tube (28), and the outer tube (20).

7. The endoscope of anyone of claims 1 through 6, **characterized in that** the intermediate tube (28) is configured, in terms of its length and wall thickness, such that the intermediate tube (28) only fills a space between the outer tube (20) and the inner tube (22) that is left free from the light guide (26).

8. The endoscope of anyone of claims 1 through 7, **characterized in that** a partially circumferential segment (30) is pushed distally onto the inner tube (22) and is arranged substantially between the outer tube (20) and the inner tube (22).

9. The endoscope of claim 8, **characterized in that** the partially circumferential segment (30) comprises a lens (32).

10. The endoscope of claim 8 or 9, **characterized in that** the partially circumferential segment (30) is beveled at its distal end.

11. The endoscope of anyone of claims 1 through 10, **characterized in that** the light guide (26) is a glass fiber bundle.

## Revendications

1. Endoscope, muni d'une tige (12), qui comporte un tube externe (20) et un tube interne (22) qui passe à l'intérieur de celui-ci, les tubes externe et interne (20, 22) étant espacés l'un de l'autre de manière radiale, de sorte que ceux-ci forment entre eux un canal (24) s'étendant axialement, dans lequel un guide de lumière (26) est inséré, **caractérisé en ce qu'**en outre un tube intermédiaire (28) résistant à la flexion est disposé entre le tube externe (20) et le tube interne (22), qui entoure au moins partiellement le tube interne (22), le tube intermédiaire (28) comportant une fente axiale.

2. Endoscope selon la revendication 1, **caractérisé en ce que** le tube intermédiaire (28) est plus court que le tube externe (20) ou le tube interne (22).

3. Endoscope selon la revendication 1 ou 2, **caractérisé en ce que** le tube intermédiaire (28) est disposé dans une zone proximale de la tige (12).

4. Endoscope selon l'une des revendications 1 à 3, **caractérisé en ce que** le tube intermédiaire (28) est biseauté à son extrémité proximale.

5. Endoscope selon l'une des revendications 1 à 4, **caractérisé en ce que** la tige (12), vue de manière radiale de l'intérieur vers l'extérieur, comporte le tube interne (22), le tube intermédiaire (28), le canal (24) et le tube externe (20).

6. Endoscope selon l'une des revendications 1 à 4, **caractérisé en ce que** la tige (12), vue de manière radiale de l'intérieur vers l'extérieur, comporte le tube interne (22), le canal (24), le tube intermédiaire (28) et le tube externe (20).

7. Endoscope selon l'une des revendications 1 à 6, **caractérisé en ce que** le tube intermédiaire (28) en ce qui concerne sa longueur et son épaisseur de paroi est façonné de sorte que le tube intermédiaire (28) n'occupe qu'un espace entre le tube externe (20) et le tube interne (22), qui est libéré par le guide de lumière (26).

8. Endoscope selon l'une des revendications 1 à 7, **caractérisé en ce qu'**un segment étendu partiellement (30) est glissé sur le tube interne (22), qui pour l'essentiel est disposé entre le tube externe (20) et le tube interne (22).

9. Endoscope selon la revendication 8, **caractérisé en ce que** le segment étendu partiellement (30) comporte une lentille (32).

10. Endoscope selon la revendication 8 ou 9, **caractérisé en ce que** le segment étendu partiellement (30) est biseauté à son extrémité distale.

11. Endoscope selon l'une des revendications 1 à 10, **caractérisé en ce que** le guide de lumière (26) est un faisceau de fibre de verre.
